# EUROPEAN PATENT SPECIFICATION

(11) **EP 0 559 825 B1**
(45) Date of publication and mention of the grant of the patent: **06.08.1997**
(21) Application number: 92902802.5
(22) Date of filing: 22.11.1991
(51) Int. Cl.: A61F 13/02, A61K 9/70, A61K 31/505

(54) **DEVICE FOR TREATING TOBACCO ADDICTION**
VORRICHTUNG ZUM BEHANDELN VON TABAKABHÄNGIGKEIT
DISPOSITIF DESTINES A TRAITER LE TABAGISME

(30) Priority: 28.11.1990 US 620064
(43) Date of publication of application: 15.09.1993
(73) Proprietor: SANO CORPORATION, Pembroke Pines, FL 33084 (US)
(72) Inventor: KENEALY, James, N., Miami, FL 33156 (US)
(74) Representative: Sternagel, Hans-Günther, Dr.
(86) International application number: US9108798
(87) International publication number: WO9209252

(56) References cited:
- EP-A- 0 341 202
- WO-A-91/08795
- GB-A- 2 222 768
- US-A- 4 940 585
- US-A- 4 971 799
- Chien, Y.W.; Transdermal Controll Systemic Medication
- Gurny,R., Teubner, A.; Dermal and Transdermal Drug Delivery

## Description

### Technical Field

The present invention relates to a device for treating tobacco craving and addiction and more particularly relates to a method and device for transdermal delivery of the drug buspirone and its derivatives and metabolites to reduce the symptoms of tobacco withdrawal and the use of tobacco.

### Background of the Invention

Tobacco smoking is a major cause of morbidity and mortality worldwide. In the United States between 350,000 and 540,000 deaths per year are attributed to tobacco smoking. Nevertheless, an estimated 54 million Americans continue to smoke. Attempts to stop smoking often are hampered by severe withdrawal symptoms including, but not limited to, drowsiness, restlessness, headache, irritability, inability to concentrate, and increased appetite. Fear of weight gain is one of the strongest disincentives to stop smoking and weight gain also is a primary cause for returning to smoking after successfully quitting.

Habitual cigarette smoking depends on an intense craving for the drug nicotine. It is nicotine that is primarily responsible for the addictive properties of tobacco. Nicotine affects both the central and peripheral nervous systems. Centrally, nicotine causes an increase in neurotransmitters including, but not limited to, dopamine, norepinephrine, beta-endorphin, vasopressin and acetylcholine. Chronic use of nicotine causes an increase in nicotine receptors in the brain. This increase in the number of binding sites may be responsible, in part, for withdrawal symptoms associated with a cessation in nicotine input. Furthermore, nicotine activates and may chronically affect central dopaminergic systems associated with reward and pleasure.

Because of the known hazards associated with tobacco, a variety of approaches have been attempted in smoking cessation programs. The psychosocial approaches include "placebo cigarettes", devices to dilute tobacco smoke, stop smoking groups, hypnosis, psychotherapy, and aversion therapy. The pharmacological approaches include nicotine administration by routes other than smoking. Oral nicotine, delivered to the blood after passage through the gastrointestinal tract, is not satisfactory because it results in irregular blood concentrations and because dosage control depends on patient compliance. Nicotine in chewing gum is delivered directly to the blood stream via the buccal cavity. Similar to oral nicotine, nicotine chewing gum results in irregular blood concentrations of nicotine. Additionally, nicotine chewing gum tastes bad, may lead to mouth ulcers and heartburn, cannot be used effectively by denture wearers, and dosage control depends on patient compliance.

Nicotine transdermal patches also are available, but in many cases can cause severe skin irritation. Other drugs that have been reported for treating tobacco addiction include clonidine and doxepin. Clonidine decreases tobacco craving, but causes sedation and hypotension. This severely limits its usefulness in a normotensive population. Doxepin, an antidepressant agent, also is an adjunct to smoking cessation. However, doxepin has anticholinergic effects which cause drowsiness and blurred vision.

Overall, smoking program quit rates average approximately 50%, but only 15% of those who quit remain abstinent for one year. Recent evidence shows the optimal program for the cessation of tobacco use should include pharmacological intervention combined with behavioral modification and group support. Statistics show a greater percentage of smokers with this therapy combination remain non-tobacco users.

Buspirone is described in U.S. Patent No. 3,717,634. Buspirone that was administered orally has been shown to reduce the craving for tobacco. Buspirone augments dopaminergic transmission by selective blockade of inhibitory dopamine autoreceptors. Buspirone is effective at low systemic blood concentrations, causes no sedation, and has no abuse potential. Orally administered buspirone, however, produces irregular blood concentrations and depends on patient compliance for its effectiveness. In addition, orally administered buspirone is poorly absorbed with only 1% to 3% of the oral dose reaching the systemic circulation.

What is needed is a method of delivering buspirone that allows for predictable blood concentrations of the drug and relies only minimally on patient compliance.

### Summary of the Invention

The present invention provides a device for treating tobacco craving and addiction by the percutaneous administration of buspirone. The buspirone is administered via a transdermal patch. The transdermal patch can be either a three-layer laminate comprising a backing layer, a buspirone loaded pressure-sensitive adhesive and a release liner. In another embodiment, a four-layer laminate comprising a backing layer, a buspirone loaded matrix layer, a pressure-sensitive adhesive layer and a release liner.

The present invention is designed to deliver buspirone over a period of time at an approximately constant rate. By practicing the present invention, an approximately constant blood concentration of the buspirone can be maintained over a period of time of 24 hours or more. For example, the patient only has to apply or have applied the transdermal patch with the buspirone therein once each 24 hour period. Thus, patient compliance required for a successful stop smoking program is minimal. Thus, an advantage of the present invention is that the transdermal patch can be administered by the patient only once every 24 hours or more. Because the buspirone blood concentrations remain relatively constant by using the present invention, the success of inhibiting tobacco craving is increased over that of prior art methods.

Accordingly, the device according to the present invention might be used for reducing the craving for tobacco.

The device according to the present invention might be used for treating tobacco addiction.

The device according to the present invention might be used to reduce the symptoms associated with withdrawal from tobacco.

The device according to the present invention might be used to avoid the increase in appetite associated with withdrawal from tobacco.

The device according to the present invention might be used to reduce the weight gain associated with withdrawal from tobacco.

It is an object of the present invention to provide the drug buspirone or its derivatives and metabolites in a transdermal device.

It is another object of the present invention to provide a transdermal device capable of sustained controlled release of buspirone over an extended period of time.

It is another object of the present invention that the transdermal device not require a discrete membrane layer for control of the buspirone flux.

It is another object of the present invention to maintain concentrations of buspirone in the systemic circulation sufficient to prevent tobacco craving for prolonged periods of time.

It is another object of the present invention to provide the patient with a pharmacological deterrent to continued tobacco use.

These and other objects, features and advantages of the present invention will become apparent after a review of the following detailed description of the disclosed embodiment and the appended claims.

### Brief Description of the Figures

Fig. 1 is a cross-sectional view of the transdermal buspirone delivery device.

Fig. 2 is cross-sectional view of another embodiment of the transdermal buspirone delivery device.

### Detailed Description of the Present Invention

The device according to the present invention might be used for treating tobacco craving and addiction, comprising maintaining a constant blood concentration of buspirone, over an extended period of time by controlled delivery of the drug from a transdermal device.

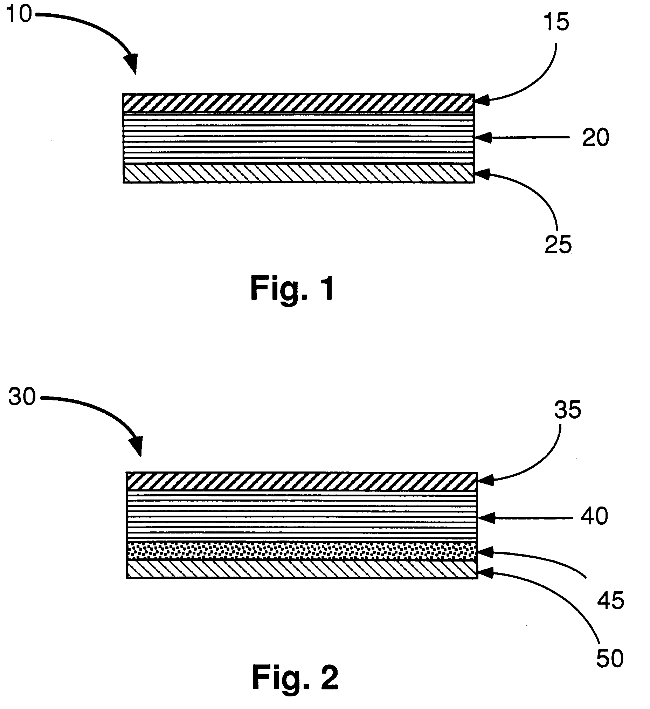

As illustrated in **FIG. 1**, the transdermal buspirone delivery device of one embodiment of the invention has an impermeable backing layer **15**, a buspirone loaded pressure-sensitive adhesive matrix layer **20**, and a release liner layer **25**, impermeable to the constituents of the pressure-sensitive adhesive layer.

As illustrated in **FIG. 2**, the transdermal buspirone delivery device **30** of another embodiment of the invention has an impermeable backing layer **35**, a buspirone loaded matrix layer **40**, a pressure-sensitive adhesive layer **45**, and a release liner layer **50** impermeable to the constituents of the matrix layer **40** and of the adhesive layer **50**.

The virtually impermeable backing layer **15** and **35** defines the top of the patch or the side furthermost away from the skin. The impermeable backing layer **15** and **35** protects the transdermal device and prevents the escape of solubilized buspirone, of constituents of the pressure-sensitive adhesive layer **20** or of the matrix layer **40** and the adhesive layer **45** into the environment.

Material used for the backing layer **15** and **35** of each embodiment should be impermeable to buspirone. The backing layer material should form a support to hold the buspirone containing matrix in comfortable contact with the patient's skin. Suitable materials for use in the backing material include, but are not limited to, dermatologically acceptable films available from 3M Corporation, Dow Chemical, or Fasson Medical Industries. A preferred backing layer is, for example: polyester film laminate sold under the trademark ScotchPak 1012 from 3M Corporation, St. Paul, MN.

The buspirone loaded adhesive layer **20** and the buspirone loaded matrix layer **40** each contain a solubilizing agent or combination of agents. Agents used to solubilize the buspirone include, but are not limited to, fatty acids such as linoleic acid and oleic acid; fatty esters such as isopropyl myristate, and isopropyl palmitate; ethers such as dipropylene glycol, dimethylisosorbide, and diethylene glycol monoethyl ether; diols such as propylene glycol, butylene glycol, and polyethylene glycol; lower alkanols containing from one to 4 carbon atoms such as ethanol and isopropyl alcohol; fatty alcohols such as oleyl, myristal, cetyl; oils such as safflower oil and maleated soybean oil; polyols such as glycerol; phospholipids such as lecithin and lecithin derivatives; polysaccharides such as hyaluronic acid; ketones such as 1-dodecylazacycloheptan-2-one (Azone, Nelson Research and Development Company, Irvine, CA) and dimethylsulfoxide. The non-adhesive matrix layer of the transdermal drug delivery device of the present invention can have between approximately 0.1% to 75% solubilizing agents.

In one embodiment of this invention **10**, solubilized or partially solubilized buspirone is dispersed in pressure-sensitive adhesives. Material used in the pressure-sensitive adhesives include, but are not limited to, natural rubber, styrene-butadiene-rubber polymers, styrene-butadiene-styrene or styrene-isoprene-styrene block copolymers, polyisoprene, polyisobutylene, butyl rubber, polyacrylates, silicone pressure-sensitive adhesives, and vinyl ether polymers. The preferred pressure-sensitive adhesives are polyacrylate, available from National Starch and Chemical Corporation, Bridgewater, N.J., polyisobutylene and butyl rubber available from Exxon Chemical Company, Houston, TX, and silicone pressure-sensitive adhesives available from Dow Corning, Midland, Michigan. The addition of tackifiers, plasticizers, fillers, pigments and antioxidants may be necessary to obtain desirable adhesive properties. The pressure-sensitive adhesive layer may be cast onto the backing layer, onto the release liner (peel strip) layer or onto an intermediary support film. The non-adhesive matrix layer of the transdermal drug delivery device of the present invention can have between approximately 20% to 99.8% pressure-sensitive adhesives.

The release liner (peel strip) layer **25** covers the surface of the pressure-sensitive adhesive during storage, protects the pressure-sensitive adhesive layer and helps maintain drug stability. The release liner (peel strip) layer may be made from any impermeable film including, but not limited to, that specified for the backing layer. One preferred class of materials for use in the release liner (peel strip) layer is polyester.

It is to be understood that the term 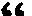 buspirone 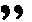 means the chemical covered by U.S. Patent No. 3,717,634 and therapeutically effective derivatives and metabolites thereof. Several of these derivatives include, but are not limited to, gepirone, ipsapirone, SM-3997 and 1-(2-pyrimidinyl)-piperazine. The buspirone has the following formula:

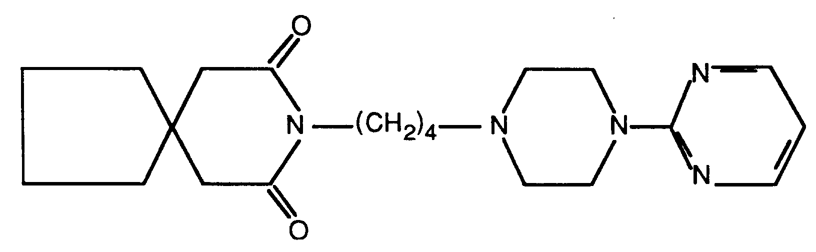

Other acid addition salts thereof are named by combining 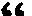 buspirone 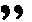 with the appropriate word to define the acid from which it is prepared as in 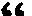 buspirone hydrochloride. 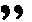 The term 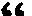 buspirone 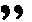 includes all salts of the base compound.

The amount of drug to be incorporated in the transdermal buspirone delivery device will vary depending on the dosage desired, the permeability of the pressure-sensitive adhesive materials, the thickness of the pressure-sensitive adhesive layer, and the length of time the transdermal delivery device is to remain on the skin and other factors. In order to achieve a therapeutic effect, the buspirone flux from the transdermal delivery device through skin should be in the range between 0.5 µg and 20 µg per cm² per hour. The rate of permeation of the drug through the pressure-sensitive adhesive material or materials can be determined readily by those skilled in the art. Thus, for example, a transdermal delivery device will deliver a maximum of 120 mg of buspirone per 24 hours and a minimum of 12 µg of buspirone per 24 hours. The non-adhesive matrix layer of the transdermal drug delivery device of the present invention can have between approximately 0.1% to 50% solubilized buspirone.

A second embodiment of the transdermal delivery device **30** comprises a backing **35** and release liner **50** as described above for embodiment **10**, a buspirone containing matrix layer **40**, and an adhesive layer **45**.

The buspirone matrix layer **40** comprises solubilized or partially solubilized buspirone dispersed in a matrix. Suitable matrix materials include, but are not limited to, polysaccharides such as starch, cellulose, hyaluronic acid, pectin, seaweed gums and vegetable gums; polypeptides such as casein, albumin, keratin and collagen; thermoplastics such as unvulconized elastomers, nylon, polyethylene (linear), polyurethane, acrylic resins, cellulose resins, and polypropylene; polyethylene glycols; polyvinylacetates; polyvinyl alcohols; and polyvinylpyrrolidones. For polyurethanes the polyether type is preferred, because in general it is more inert than polyester types, and thus more appropriate for medical use. Polymers of this type are available from B. F. Goodrich Company, Brecksville, OH.

The pressure-sensitive adhesive layer **45** contains a dermatologically acceptable adhesive or adhesives. A suitable adhesive is 3M-1778 double sided adhesive from 3M Corporation, St. Paul, MN.

To prepare a transdermal buspirone delivery device, dissolve or disperse the buspirone in the solubilizing agent or agents and the polymer adhesive or adhesives. The percentage buspirone in this solution may be varied according to the desired loading of the finished matrix. The buspirone content of the finished pressure-sensitive adhesive layer may vary from 0.1% to 50%. For example, where it is desired to release between 12 µg to 120 mg of buspirone in a 24 hour period, the preferred buspirone load in the adhesive matrix is 0.1% to 25%.

The matrix may be processed by utilizing the art known in casting (pouring into a mold or on a moving flat surface), coating, extrusion, hot melt applications, radiation curing or other methods known in the art. The matrix will typically have a thickness in the range of 10 to 1400 microns. For a given total buspirone load, the percentage loading may be varied by varying the matrix thickness.

The matrix is then laminated to the backing layer and to the release liner (peel strip) layer by techniques known in the art, to form the multilayered structures shown in **FIGS. 1** and **2**. Patches of the desired size are punched out from the laminate by techniques known in the art. Punched patches can range from approximately 1 to 200 cm². The more preferable patch size is from 2 to 60 cm². The size of the patch will vary according to the amount of buspirone to be delivered over a 24 hour period. To prevent contamination and to maintain the stability of the buspirone and the adhesive, the punched transdermal buspirone delivery devices are sealed in individual pouches or other suitable materials until used. It should be noted that the transdermal patch which is contemplated as the present invention can be used anywhere on the body where the patch can be applied to the skin.

This invention is further illustrated by the following examples, which are not construed in any way as imposing limitations upon the scope thereof. On the contrary, it is to be clearly understood that resort may be had to various embodiments, modifications, and equivalents thereof which, after reading the description herein, may suggest themselves to those skilled in the art without departing from the scope of the appended claims.

### Example 1

The present invention includes the administration of buspirone in a transdermal delivery device. In one embodiment of a transdermal device according to the present invention, buspirone, 5% by weight, is blended with 5% oleic acid and 10% propylene glycol. The buspirone blend is added to 10% silicone Medical Fluid 360 and 70% silicone pressure-sensitive adhesive and mixed until homogeneous. The mixture is then coated uniformly onto a layer of a PTFE coated polyester release liner (ScotchPak 1022, 3M Corporation, St. Paul, MN). The preparation is dried at a temperature between 15 C° and 30 C° until the solvents evaporate and the system has pressure-sensitive adhesive properties. A backing material is then laminated to the buspirone loaded pressure-sensitive adhesive layer/release liner (peel strip) layer to form a triple laminate of backing layer/buspirone loaded pressure-sensitive adhesive layer/release liner (peel strip) layer. The laminate is then punched into units of 10 cm², containing 0.5 mg/cm² of buspirone and the transdermal buspirone delivery devices are stored in individual packets for use within 24 months. For use, one transdermal buspirone delivery device is removed from its packet, the release liner (peel strip) layer is removed and discarded, and the buspirone loaded pressure-sensitive adhesive is applied firmly to the patient's arm. The transdermal buspirone delivery device is left in place for 24 hours.

### Example 2

In another embodiment, buspirone, 5% by weight, is blended with 5% dimethylisosorbide and 10% dipropylene glycol until homogeneous. The blend is heated and is added to and mixed with molten polyvinylacetate which has been melted slowly to obtain a working viscosity of 10,000 to 20,000 cps. The matrix layer mixture is slot die coated onto the backing layer. To do this, the mixture is flowed through a slot to form a thin coat of a desired thickness which is deposited onto a layer of polyester film laminate backing layer (ScotchPak 1012, 3M Corporation, St. Paul, MN). A layer of double-sided pressure-sensitive polyacrylate adhesive such as 3M-1778 and a layer of silicone coated polyester release liner are laminated to the face of the matrix facing away from the backing layer to form a quadruple laminate of backing layer/buspirone loaded matrix layer/pressure-sensitive adhesive layer/release liner (peel strip) layer. The laminate is then punched into transdermal units of 10 cm², containing 0.5 µg/cm² of buspirone and stored in individual packets for use within 24 months. For use, one transdermal buspirone delivery device is removed from its packet, the release liner (peel strip) layer is removed and discarded, and the pressure-sensitive adhesive layer is applied firmly to the patient's arm. The transdermal buspirone delivery device is left in place for 24 hours.

While the invention has been described in detail and with reference to specific embodiments thereof, it will be apparent to one skilled in the art that various changes and modifications can be made therein without departing from the scope thereof.

## Claims

1. A transdermal drug delivery device (10) comprising a laminate of
(a) a pressure-sensitive adhesive layer (20) having a first and a second face and containing a drug,
(b) a backing layer (15) substantially impermeable to the drug and contacting the first face of the pressure-sensitive adhesive layer, and
(c) a release liner layer (25) contacting the second face of the pressure-sensitive adhesive layer (20) ,
**characterized in that**
the drug is solubilized buspirone and the pressure-sensitive adhesive layer (20) contains a solubilizing agent or a combination of agents.

2. A transdermal drug delivery device (30) comprising a laminate of
(a) a non-adhesive matrix layer (40) having a first and second face and containing a drug,
(b) a backing layer (35) substantially impermeable to the drug and contacting the first face of the non-adhesive matrix layer (40),
(c) a pressure-sensitive adhesive layer (45) having a first and second face, the first face of the pressure-sensitive layer (45) contacting the second face of the non-adhesive matrix layer (40), and
(d) a release liner layer (50) contacting the second face of the pressure-sensitive adhesive layer (45)
**characterized in that**
the drug is solubilized buspirone and the matrix layer (40) contains a solubilizing agent or a combination of agents.

3. The transdermal drug delivery device (10) of claim 1
**characterized in that**
the pressure-sensitive adhesive layer (20) comprises
(a) 0.1 to 50% by weight solubilized buspirone.
(b) 0.1 to 75% by weight solubilizing agent and
(c) 99.8 to 20% pressure-sensitive adhesives.

4. The transdermal drug delivery device (30) of claim 2
**characterized in that**
the non-adhesive matrix layer (40) comprises
(a) 0.1 to 50% by weight of solubilized buspirone,
(b) 0.1 to 75% by weight of solubilizing agent.

5. The transdermal drug delivery device (10, 30) of any of claims 1 to 4
**characterized in that**
the solubilizing agent is selected from fatty acids, fatty esters, ethers, diols, fatty alcohols, oils, polyols, phospholipids, polysaccharides, and ketones.

6. The transdermal drug delivery device (30) of claim 2
**characterized in that**
the non-adhesive matrix materials are selected from polysaccharides, polypeptides, thermoplastics, polyethylene glycols, polyvinylacetates, polyvinylalcohols, and polyvinylpyrrolidones.

7. The transdermal drug delivery device (10, 30) of any of claims 1 to 6
**characterized in that**
the pressure-sensitive adhesive are selected from natural rubber, styrene-butadiene rubber polymers, styrene-butadiene-styrene or styrene-isoprene-styrene block copolymers, polyisoprene, polyisobutylene, butyl rubber, polyacrylates, silicone pressure-sensitive adhesives, and vinyl ether polymers.

8. The transdermal drug delivery device (10, 30) of any of claims 1 to 7
**characterized in that**
the release of buspirone is in the range between 0.5 µg and 20 µg per cm² per hour.

9. The transdermal drug delivery device (30) of claim 2
**characterized in that**
the non-adhesive matrix layer (40) has a thickness in the range of 10 to 1400 µm.

10. The transdermal drug delivery device (10, 30) according to any of claims 1-9 for use in treating tobacco craving and addiction.

## Patentansprüche

1. Transdermale Vorrichtung (10) zur Verabreichung von Wirkstoff, umfassend ein Laminat aus
(a) einer Haftklebeschicht (20), die eine erste und zweite Fläche aufweist und einen Wirkstoff enthält,
(b) eine Trägerschicht (15), die im wesentlichen für den Wirkstoff undurchlässig ist und die erste Fläche der Haftklebeschicht berührt, und
(c) eine Trennschicht (25), die die zweite Fläche der Haftklebeschicht (20) berührt,
**dadurch gekennzeichnet**,
daß der Wirkstoff gelöstes Buspiron ist und die Haftklebeschicht (20) einen Lösungsvermittler oder eine Kombination von Vermittlern enthält.

2. Transdermale Vorrichtung (30) zur Verabreichung von Wirkstoff, umfassend ein Laminat aus
(a) einer nichthaftenden Matrixschicht (40), die eine erste und zweite Fläche aufweist und einen Wirkstoff enthält,
(b) eine Trägerschicht (35), die im wesentlichen für den Wirkstoff undurchlässig ist und die erste Fläche der nichthaftenden Matrixschicht (40) berührt,
(c) eine Haftklebeschicht (45) mit einer ersten und zweiten Fläche, wobei die erste Fläche der Haftklebeschicht (45) die zweite Fläche der nichthaftenden Matrixschicht (40) berührt, und
(d) eine Trennschicht (50), die die zweite Fläche der Haftklebeschicht (45) berührt,
**dadurch gekennzeichnet**,
daß der Wirkstoff gelöstes Buspiron ist und die Matrixschicht (40) einen Lösungsvermittler oder eine Kombination von Vermittlern enthält.

3. Transdermale Vorrichtung (10) zur Verabreichung von Wirkstoff nach Anspruch 1,
**dadurch gekennzeichnet**,
daß die Haftklebeschicht (20)
(a) 0,1-50 Gew.-% gelöstes Buspiron.
(b) 0,1-75 Gew.-% Lösungsvermittler und
(c) 99,8-20 Gew.-% Haftklebstoffe
enthält.

4. Transdermale Vorrichtung (30) zur Verabreichung von Wirkstoff nach Anspruch 2,
**dadurch gekennzeichnet**,
daß die nichthaftende Matrixschicht (40)
(a) 0,1-50 Gew.-% gelöstes Buspiron, und
(b) 0,1-75 Gew.-% Lösungsvermittler
enthält.

5. Transdermale Vorrichtung (10,30) zur Verabreichung von Wirkstoff nach einem der Ansprüche 1-4,
**dadurch gekennzeichnet**,
daß der Lösungsvermittler ausgewählt ist aus Fettsäuren, Fettsäureestern, Ethern, Diolen, Fettalkoholen, Ölen, Polyolen, Phospholipiden, Polysacchariden und Ketonen.

6. Transdermale Vorrichtung (30) zur Verabreichung von Wirkstoff nach Anspruch 2,
**dadurch gekennzeichnet**,
daß die nichthaftenden Matrixmaterialien ausgewählt sind aus Polysacchariden, Polypeptiden, thermoplastischen Materialien, Polyethylenglykolen, Polyvinylacetaten, Polyvinylalkoholen und Polyvinylpyrrolidonen.

7. Transdermale Vorrichtung (10,30) zur Verabreichung von Wirkstoff nach einem der Ansprüche 1-6,
**dadurch gekennzeichnet**,
daß der Haftklebstoff ausgewählt ist aus natürlichem Kautschuk, Styrol-Butadien-Kautschukpolymeren, Styrol-Butadien-Styrol- oder Styrol-Isopren-Styrol-Blockcopolymeren, Polyisopren, Polyisobutylen, Butylkautschuk, Polyacrylaten, Siliconhaftklebstoffen und Vinyletherpolymeren.

8. Transdermale Vorrichtung (10,30) zur Verabreichung von Wirkstoff nach einem der Ansprüche 1-7,
**dadurch gekennzeichnet**,
daß die Abgabe von Buspiron im Bereich zwischen 0,5 und 20 µg pro cm² pro Stunde liegt.

9. Transdermale Vorrichtung (30) zur Verabreichung von Wirkstoff nach Anspruch 2,
**dadurch gekennzeichnet**,
daß die nichthaftende Matrixschicht (40) eine Dicke im Bereich von 10-1400 µm aufweist.

10. Transdermale Vorrichtung (10,30) zur Verabreichung von Wirkstoff nach einem der Ansprüche 1-9 zur Verwendung bei der Behandlung von Tabakabhängigkeit und Sucht.

## Revendications

1. Dispositif de délivrance transdermique de médicament (10) comprenant un feuilleté d'
(a) une couche autocollante sensible à la pression (20) ayant une première et une seconde face et contenant un médicament,
(b) une couche dorsale (15) essentiellement imperméable au médicament et en contact avec la première face de la couche autocollante sensible à la pression, et
(c) une couche de couverture de délivrance (25) en contact avec la seconde face de la couche autocollante sensible à la pression (20),
caractérisé en ce que
le médicament est la buspirone solubilisée et la couche autocollante sensible à la pression (20) contient un agent de dissolution ou une combinaison d'agents.

2. Un dispositif de délivrance transdermique de médicament (30)comprenant un feuilleté d'
(a) une couche de matrice non-autocollante (40) ayant une première et une seconde face et contenant un médicament,
(b) une couche dorsale (35) essentiellement imperméable au médicament et en contact avec la première face de la couche de matrice non-autocollante (40),
(c) une couche autocollante sensible à la pression (45) ayant une première et une seconde face, la première face de la couche sensible à la pression (45) étant en contact avec la seconde face de la couche de matrice non-autocollante (40), et
(d) une couche de couverture de délivrance (50) en contact avec la seconde face de la couche autocollante sensible à la pression (40),
caractérisé en ce que
le médicament est la buspirone solubilisée et la couche de matrice (40) contient un agent de dissolution ou une combinaison d'agents.

3. Dispositif de délivrance transdermique de médicament (10) selon la revendication 1
caractérisé en ce que
la couche autocollante sensible à la pression (20) comprend :
(a) 0,1 à 50 % en poids de buspirone solubilisée,
(b) 0,1 à 75 % en poids d'agent de dissolution et
(c) 99,8 à 20 % d'adhésifs sensibles à la pression.

4. Dispositif de délivrance transdermique de médicament (30) selon la revendication 2
caractérisé en ce que
la couche de matrice non-autocollante (40) comprend
(a) 0,1 à 50 % en poids de buspirone solubilisée,
(b) 0,1 à 75 % en poids d'agent de dissolution.

5. Dispositif de délivrance transdermique de médicament (10, 30) selon l'une quelconque des revendications 1 à 4
caractérisé en ce que
l'agent de dissolution est choisi parmi les acides gras, les esters d'acide, les esters, les diols, les alcools gras, les huiles, les polyols, les phospholipides, les polysaccharides, et les cétones.

6. Dispositif de délivrance transdermique de médicament (30) selon la revendication 2
caractérisé en ce que
les matériaux de matrice non-autocollants sont choisis parmi les polysaccharides, les polypeptides, les thermoplastiques, les polyéthylène-glycols, les polyvinylacétates, les polyvinylalcools, et les polyvinylpyrrolidones.

7. Dispositif de délivrance transdermique de médicament (10, 30) selon l'une quelconque des revendications 1 à 6
caractérisé en ce que
les adhésifs sensibles à la pression sont choisis parmi la caoutchouc naturel, les polymères à base de caoutchouc styrène-butadiène, les copolymères à base de blocs de styrène-butadiène-styrène ou styrène-isoprène-styrène, le polyisoprène, le polyisobutylène, le caoutchouc à base de butyle, les polyacrylates, les adhésifs sensibles à la pression à base de silicone, et les polymères à base de vinyléther.

8. Dispositif de délivrance transdermique de médicament (10, 30) selon l'une quelconque des revendications 1 à 7
caractérisé en ce que
la délivrance de buspirone s'effectue dans une variation entre 0,5 µg et 20 µg par cm² par heure.

9. Dispositif de délivrance transdermique de médicament (30) selon la revendication 2
caractérisé en ce que
la couche de matrice non-autocollante (40) a une épaisseur dans la gamme de 10 à 1400 µm.

10. Dispositif de délivrance transdermique de médicament (10, 30) selon l'une quelconque des revendications 1 à 9 pour l'utilisation dans le traitement du besoin en tabac et du tabagisme.
